# DEMANDE DE BREVET EUROPEEN

(11) **EP 1 986 031 A2**
(43) Date de publication de la demande: **29.10.2008**
(21) Numéro de dépôt: 08159710.6
(22) Date de dépôt: 11.07.2003
(51) Int. Cl.: G02B 23/26, G01N 21/64, A61B 1/07, A61B 5/00

(54) **Procédé et appareillage d'imagerie de fluorescence fibrée haute résolution**

(30) Priorité: 18.07.2002 FR 0209099; 11.03.2003 FR 0302972
(62) Demande divisionnaire de: 03755595.0
(71) Demandeur: Mauna Kea Technologies, 75010 Paris (FR)
(72) Inventeur: Genet, Magalie, 78280, GUYANCOURT (FR); Viellerobe, Bertrand, 94130, NOGENT SUR MARNE (FR); Berier, Frédéric, 29810, PLOUARZEL (FR); Clade, Sophie, 75016, PARIS (FR)
(74) Mandataire: Pontet, Bernard

(57) **Abrégé**

Le procédé utilise un guide d'image fait de plusieurs milliers de fibres optiques, un signal d'excitation étant émis par une source, dévié et injecté tour à tour dans l'une des fibres dudit guide, chaque point d'excitation du tissu en sortie de fibre émettant en retour un signal de fluorescence collecté par ladite fibre, puis détecté et numérisé pour former un élément d'image. Selon l'invention, le procédé prévoit de produire un faisceau divergent en sortie de fibre susceptible d'exciter un microvolume du tissu depuis la surface. Selon l'invention, le procédé est caractérisé en ce que l'on dévie le signal d'excitation à une vitesse correspondant à l'acquisition d'un nombre d'images par seconde suffisant pour une utilisation en temps réel et en ce que l'on détecte le signal de fluorescence à une fréquence de détection correspondant à une fréquence minimale d'échantillonnage des fibres une à une.

Grâce à l'invention, on peut réaliser une image in vivo in situ et en temps réel.

## Description

La présente invention concerne un procédé et un appareillage d'imagerie de fluorescence fibrée haute résolution. Le domaine d'application visé est plus particulièrement celui de l'imagerie in vivo et in situ.

La fluorescence observée peut provenir d'un composé exogène (typiquement un marqueur injecté) ou endogène (présent dans la cellule) d'un tissu biologique.

Plus particulièrement, un procédé d'imagerie confocale est du type consistant à balayer point par point un tissu dans un plan subsurfacique, chaque point correspondant à un signal d'excitation émis par une source continue, dévié et injecté tour à tour dans une fibre optique d'un faisceau de fibres optiques, puis focalisé à la sortie de ladite fibre dans ledit plan, chaque point émettant en retour un signal de fluorescence collecté par ladite fibre optique, puis détecté et numérisé pour former un élément d'image.

Le caractère confocal est obtenu en utilisant le même chemin optique, notamment la même fibre optique servant de filtrage spatial, pour transporter le signal d'excitation et le signal de fluorescence émis en réaction, et en utilisant un système optique permettant de conjuguer le point de focalisation dans le tissu avec ladite fibre optique.

L'appareillage d'imagerie confocale correspondant comprend le faisceau de fibres optiques souples (appelé couramment guide d'image) avec, à son extrémité proximale :
- la source émettant en continu ou en mode pulsé à la longueur d'onde d'excitation d'un ou plusieurs fluorophores ciblés, typiquement une source laser ;
- des moyens de balayage rapide dans le temps du faisceau d'excitation produit par la source en lignes et en colonnes dans un plan XY correspondant à la section d'entrée du guide d'image ;
- des moyens d'injection du faisceau d'excitation dans l'une des fibres optiques ;
- des moyens de détection du signal de fluorescence ; et
- des moyens de commande, notamment des moyens de balayage.

Des moyens étant en outre prévus pour permettre la réalisation et la visualisation d'une image à partir des signaux détectés successivement sur chaque fibre.

A l'extrémité distale du guide d'image, se trouve une tête optique, destinée à être mise en contact avec le tissu observé, permettant de focaliser le faisceau d'excitation à une profondeur donnée (quelques dizaines de µm) et donc de réaliser une image subsurfacique.

En pratique, ce type d'appareillage présente notamment les avantages suivants :
- du côté distal du guide d'image se trouve uniquement une optique de focalisation, qui est moins fragile et moins coûteuse qu'une tête optique intégrant des moyens de balayage, le remplacement de la tête optique pouvant être envisagé indépendamment des moyens de balayage ; par ailleurs la tête est miniaturisable, ce qui présente un intérêt en endoscopie et également, d'une manière générale, pour augmenter la précision de positionnement ;
- un guide d'image fait de fibres optiques souples, sert de bras d'accès au site à observer, ce qui est important pour une application in situ.

Dans l'état de la technique, on a décrit les procédés et appareillages de fluorescence suivants.

Dans un article paru dans "Applied Optics", Vol.38, No. 34, décembre 1999, pages 7133-7144, est présenté un micro-endoscope confocal mettant en oeuvre un faisceau de fibres optiques souples muni à son extrémité distale d'une tête optique miniature de focalisation; il est prévu de balayer le faisceau de fibres optiques en lignes, l'appareillage comprenant une fente de détection ainsi qu'un détecteur linéaire CCD de type à transfert de charges. Un appareillage de ce type permet d'obtenir 4 images/s, ce qui est trop lent pour une imagerie in situ tributaire des bougés du sujet et de l'opérateur. Par ailleurs, le fait de balayer en lignes et non point par point dégrade le caractère confocal et conduit à une image moins bien résolue.

Dans un article paru dans "Optics Communications", 188 (2001), pages 267-273, est présenté le couplage d'un microscope confocal de table à balayage laser avec un faisceau de fibres optiques souples muni à son extrémité distale d'une tête optique miniature. Le but recherché est de rendre le microscope compatible avec un endoscope. Le balayage est réalisé fibre à fibre mais le microscope de table utilisé est de conception classique, conçu pour visualiser un échantillon fixe sans soucis du temps d'obtention d'une image. Un temps de pose de 2 secondes est avancé dans cet article, beaucoup trop long pour une imagerie in situ, en temps réel.

La présente invention a pour but de proposer un procédé et un appareillage d'imagerie de fluorescence fibrée haute résolution du type utilisant un guide d'image fait de fibres optiques balayées une à une par un faisceau d'excitation émis en continu, lesdits procédé et appareillage permettant la visualisation en temps réel d'un site in vivo in situ, c'est-à-dire capable de fournir un nombre suffisant d'images point à point par seconde sans être tributaire des bougés du sujet et du praticien pour permettre notamment un examen assez rapide.

La présente invention a également pour objet de proposer un procédé et un appareillage qui, d'une manière générale, optimisent la qualité de chaque image afin notamment d'obtenir une excellente résolution latérale et axiale.

Selon l'invention, il est proposé un procédé pour réaliser une image de fluorescence haute résolution à l'aide d'un guide d'image fait de plusieurs milliers de fibres optiques, un signal d'excitation étant émis par une source continue, dévié et injecté tour à tour dans l'une des fibres optiques dudit guide d'image et un signal de fluorescence émis en réaction étant collecté par la même fibre optique que celle utilisée pour l'excitation, puis détecté et numérisé pour former un élément d'image, caractérisé en ce que l'extrémité des fibres est destinée à être placée à nue directement en contact de la surface du tissu à imager, chaque fibre étant adaptée à produire un faisceau divergent susceptible d'exciter un micro-volume du tissu situé de la surface jusqu'à une profondeur maximale dépendante notamment du diamètre de coeur des fibres optiques et en ce que l'on dévie le signal d'excitation à une vitesse correspondant à l'acquisition d'un nombre d'images par seconde suffisant pour une utilisation en temps réel et en ce que l'on détecte le signal de fluorescence à une fréquence de détection correspondant à une fréquence minimale d'échantillonnage des fibres une à une.

Ce procédé ne prévoit pas le balayage d'un signal qui est focalisé en sortie de chaque fibre mais le balayage d'un signal divergent en sortie de chaque fibre. La non focalisation du signal en sortie de fibre permet d'obtenir des images d'un volume situé juste sous la surface du tissu qui sont exploitables et intéressantes d'un point de vue médical notamment. Ces images ne sont pas « confocales » car elles ne proviennent pas d'un plan de coupe subsurfacique balayé point à point, mais des images que l'on peut toutefois qualifier de « hautement résolues » car provenant du balayage tour à tour de microvolumes situés directement sous la surface et d'un filtrage spatial du signal de fluorescence émis par chaque microvolume par la même fibre que celle ayant servie à l'excitation. Le principal avantage d'un tel appareillage réside dans le fait que pour une application endoscopique, le diamètre de la sonde endoscopique peut être très petit dépendant uniquement du diamètre du guide d'image et donc de son nombre de fibres optiques. Cela permet d'envisager des domaines d'applications, comme par exemple le domaine de la neurologie, où la taille de la sonde endoscopique est un facteur critique en s'affranchissant des problèmes inhérents à la miniaturisation de la tête optique de focalisation.

La présente invention propose une solution pour réaliser une image in vivo in situ en temps réel, que ce soit pour un procédé avec ou sans focalisation en sortie de fibre. L'invention est basée sur le respect de l'échantillonnage des fibres (selon le critère de Shannon) qui permet d'obtenir et de reconstruire une image point à point correspondant bien à chaque fibre. Cela permet de ne pas perdre d'information en échantillonnant l'ensemble des fibres une à une tout en respectant un nombre moyen minimal d'images par seconde, à savoir en pratique au minimum 12 images par secondes pour un mode maximal de 640 x 640 pixels. Le choix de la fréquence de détection (bande passante du détecteur) en fonction de cet échantillonnage minimal permet ensuite pour chaque fibre de détecter le plus grand nombre possible de photons de fluorescence.

Ainsi, selon un mode de réalisation possible, mettant en oeuvre un guide d'image d'environ 30 000 fibres optiques souples, la fréquence d'échantillonnage et la bande passante du système de détection (une photodiode à avalanche ou équivalent) sont fixés sensiblement à 1,5 MHz, correspondant environ à 12 pixels par fibre, permettant alors d'obtenir au minimum les 12 images/s en mode maximal 640 x 640 pixels.

En pratique, selon un mode de réalisation avantageux, permettant un balayage rapide approprié des fibres pour obtenir une image en temps réel, la déviation du faisceau est réglée en déterminant une fréquence de résonance rapide d'un miroir résonnant « ligne » et une fréquence de résonance lente d'un miroir galvanométrique « trame ».

Selon l'invention, la durée de détection d'un flux en provenance d'une fibre est limitée dans le temps et très courte pour respecter l'acquisition d'une image en temps réel. Selon l'invention, on a donc en outre prévu des moyens d'optimisation pour collecter et détecter le maximum de flux en provenance de l'échantillon pendant cette durée de détection limitée, notamment :
- on utilise des moyens optiques de déviation, d'injection, le cas échéant de focalisation et de détection présentant un degré d'achromaticité dépendant de l'écart en longueur d'onde entre la longueur d'onde d'excitation et la longueur d'onde maximale de fluorescence, ainsi que de la largeur spectrale du signal de fluorescence émis ; cela permet avantageusement d'optimiser le flux de fluorescence collecté sur toute la bande spectrale de fluorescence émise ;
- on choisit, le cas échéant, une ouverture numérique de l'optique de focalisation sur le tissu comprise sensiblement entre 0,5 et 1, permettant d'obtenir un bon compromis entre la taille de champ et la quantité de photons collectée ; et
- on choisit un détecteur ayant une efficacité quantique aux longueurs d'ondes de fluorescence à détecter d'au moins 50%.

Selon l'invention, le traitement d'image réalisé ensuite sur le flux détecté est également optimisé, pour obtenir une image de très bonne qualité à partir de ce flux limité de photons détecté. Cette optimisation est réalisée de la manière suivante.

Une série d'étapes est réalisée préalablement à l'acquisition d'images en temps réel :
- une étape de détection de l'emplacement de chaque fibre d'un ensemble choisi de fibres destinés à être utilisées (soit l'ensemble du guide d'image soit un sous-ensemble choisi) ; cette étape est à réaliser au moins à chaque changement de guide d'image ;
- une étape de calibration du taux d'injection dans chaque fibre, c'est à dire de définition d'un taux d'injection propre à chaque fibre ; et
- une étape de détection de l'image de fond (sans échantillon).

En fonctionnement, l'optimisation du traitement d'image comprend notamment les étapes consistant, après numérisation du signal détecté :
- à définir le flux réel collecté par chaque fibre c'est-à-dire ne provenant que de l'échantillon, après correction en fonction du taux d'injection propre de la fibre et de la soustraction de l'image du fond, de manière à obtenir un signal corrigé;
- puis à effectuer une reconstruction de l'image à partir de ce signal corrigé, avec pour but notamment de transformer une image présentant une mosaïque de fibres en une image sans fibres apparentes.

Selon l'invention, ces deux dernières étapes doivent être réalisables en temps réel. Pour ce qui est de la correction du signal, celle-ci peut se faire en temps réel grâce à un traitement adapté à la structure du signal observé et un algorithme optimisé. Pour ce qui est de la reconstruction de l'image, elle peut se faire grâce au choix d'un nombre d'opérations par pixel réalisables en temps réel permettant d'obtenir le résultat recherché en terme de qualité d'image. Un filtrage passe-bas Gaussien représente un bon compromis entre la complexité du traitement, la qualité du résultat et le temps de calcul.

Pour gagner du temps et augmenter la complexité du traitement correspondant à la reconstruction de l'image, on peut aussi augmenter la capacité de traitement du matériel, par exemple en utilisant une carte de traitement spécifique et/ou une architecture parallèle comme un multiprocesseur.

La présente invention propose un appareillage pour la mise en oeuvre du procédé selon l'invention, comprenant :
- le guide d'image ;
- la source émettant en continu à la longueur d'onde d'excitation d'au moins un fluorophore ciblé,
- des moyens de balayage et d'injection fibre après fibre du faisceau d'excitation produit par la source dans un plan XY correspondant à la section d'entrée du guide d'image ;
- des moyens de séparation de la longueur d'onde d'excitation et des longueurs d'onde de fluorescence ;
- des moyens de détection du signal de fluorescence ; et
- des moyens de traitement du signal détecté permettant la réalisation d'une image ;
caractérisé en ce que l'extrémité de chaque fibre est adaptée à produire un faisceau qui est divergent et est destinée à être placée à nue directement au contact de la surface du tissu à observer ;
et en ce que les moyens de balayage sont adaptés à déplacer le faisceau d'excitation à une vitesse correspondant à l'obtention d'une image en temps réel et les moyens de détection présentent une bande passante dont la fréquence est fixée en fonction de la fréquence minimale d'échantillonnage des fibres une à une.

La présente invention sera mieux comprise et d'autres avantages apparaîtront à la lumière de la description qui va suivre d'un exemple de réalisation, description faite en référence aux dessins annexés sur lesquels :
- la figure 1 représente schématiquement un exemple de réalisation d'appareillage d'imagerie confocale de fluorescence comprenant une tête de focalisation ;
- la figure 2 est un schéma fonctionnel de l'appareillage de la figure 1 ;
- la figure 3 représente schématiquement un exemple de réalisation d'appareillage d'imagerie de fluororescence haute résolution ; et
- la figure 4 est un schéma fonction de l'appareillage de la figure 3.

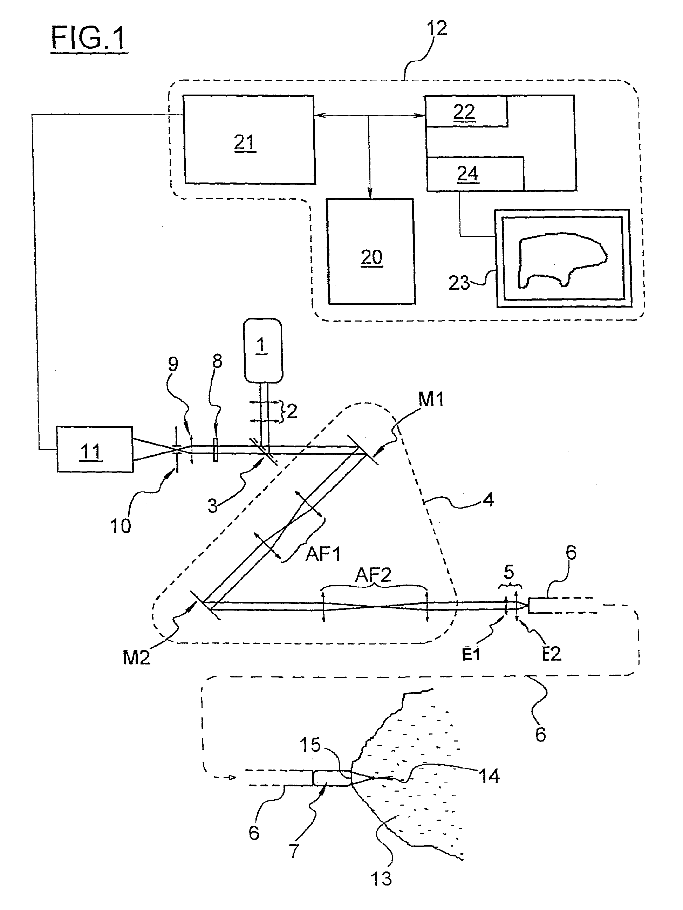

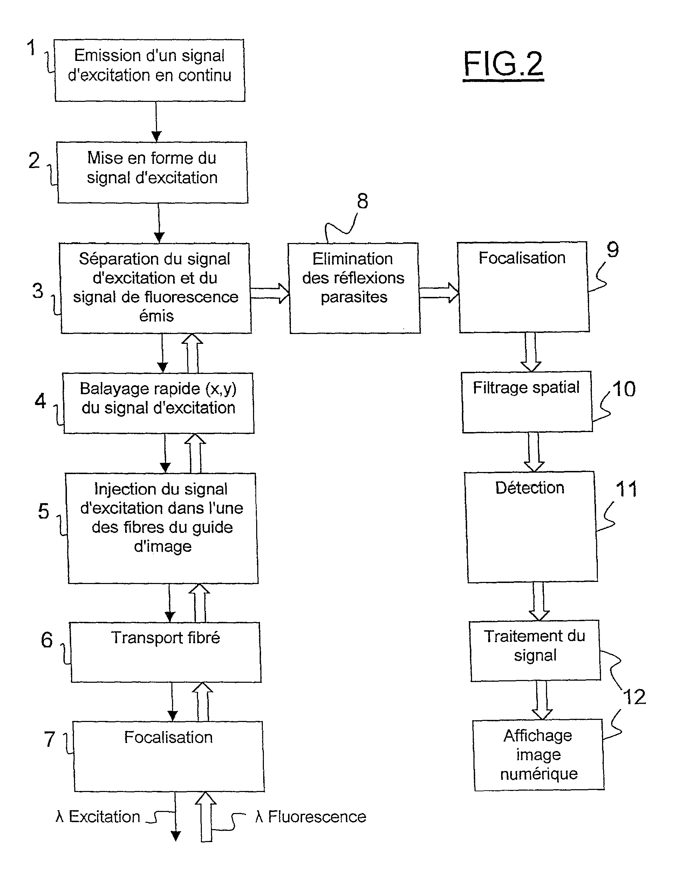

Selon l'exemple choisi et représenté sur les figures 1 et 2, l'appareillage comporte :
- une source lumineuse 1 ;
- des moyens 2 de mise en forme du faisceau d'excitation ;
- des moyens 3 de séparation de longueurs d'onde ;
- des moyens 4 de balayage ;
- des moyens 5 d'injection de faisceau ;
- un guide d'image 6 constitué de fibres optiques souples ;
- une tête optique de focalisation 7 ;
- des moyens 8 de réjection du faisceau d'excitation ;
- des moyens 9 de focalisation du signal de fluorescence ;
- des moyens 10 de filtrage spatial du signal de fluorescence ;
- des moyens 11 de détection du signal de fluorescence ; et
- des moyens 12 de traitement électronique et informatique du signal de fluorescence détecté et de visualisation.

Ces différents éléments sont détaillés ci-après.

La source lumineuse 1 est un laser émettant à une longueur d'onde d'excitation permettant d'exciter une grande gamme de fluorophores, par exemple 488 nm. Pour optimiser l'injection dans l'une des fibres du guide d'image 6, le faisceau d'excitation est circulaire pour pouvoir injecter une fibre de section également circulaire et, pour optimiser le taux d'injection, le laser est de préférence monomode longitudinal pour présenter le meilleur front d'onde possible pour l'injection dans une fibre optique faiblement multimode. Le laser émet de manière continue ou de manière pulsée, et de manière stable (bruit le plus faible possible, <1%). La puissance en sortie disponible est de l'ordre de 20mW. A titre d'exemples, on peut utiliser un laser à puits quantiques (VCSEL), un laser solide pompé par diode, une diode laser ou encore un laser à gaz tel que l'Argon.

En sortie de la source 1, sont placés les moyens 2 de mise en forme du faisceau laser d'excitation. Ils sont constitués d'un système optique afocal de grandissement différent de 1, composé de deux lentilles L1 et L2 qui permettent de modifier le diamètre du faisceau laser. Le grandissement est calculé de sorte que le diamètre du faisceau soit adapté aux moyens d'injection 5 dans une fibre.

Le faisceau laser d'excitation remis en forme est ensuite dirigé vers les moyens 3 prévus pour séparer les longueurs d'ondes d'excitation et de fluorescence. Il s'agit par exemple d'un filtre dichroïque ayant une efficacité de transmission de 98 à 99 % de la longueur d'onde d'excitation et qui réfléchit donc sensiblement les autres longueurs d'onde. Le signal de fluorescence, empruntant au retour le même chemin optique que le signal d'excitation (caractère confocal), sera ainsi envoyé pratiquement totalement vers la voie de détection (8-11). Les moyens de réjection 8 placés sur la voie de détection servent à éliminer totalement les 1 à 2 % de réflexions parasites à la longueur d'onde d'excitation 488 nm qui passent vers la voie de détection (par exemple un filtre de réjection à 488 nm, un filtre passe bande ne permettant par exemple qu'une transmission entre 500 et 600nm, ou un filtre passe-haut qui permet une transmission au dessus de 500 nm).

Les moyens de balayage 4 reprennent ensuite le faisceau d'excitation. Selon l'exemple choisi et représenté sur la figure 1, ces moyens comprennent un miroir M1 résonant à 4 KHz servant à dévier le faisceau horizontalement et donc à réaliser les lignes de l'image, d'un miroir M2 galvanométrique à 15 Hz servant à dévier le faisceau verticalement et donc à réaliser la trame de l'image ; et de deux systèmes afocaux de grandissement unitaire, AF1 situé entre les deux miroirs et AF2 situé après le miroir M2, ces systèmes afocaux étant utilisés pour conjuguer les plans de rotation des deux miroirs M1 et M2 avec le plan d'injection dans l'une des fibres. Selon l'invention, la vitesse de balayage est déterminée pour permettre une observation des tissus in vivo in situ en temps réel. Pour cela, le balayage doit être suffisamment rapide pour qu'il y ait au moins 12 images / s affichées à l'écran pour un mode d'affichage de 640 x 640 pixels correspondant au mode le plus lent. Pour les modes d'affichage ayant moins de pixels, le nombre d'images acquises par seconde sera ainsi toujours supérieur à 12 images / s. En variante, les moyens de balayage peuvent comprendre notamment un miroir rotatif, des composants intégrés de type MEMs (miroirs de balayage X et Y), ou un système acousto-optique.

Le faisceau d'excitation dévié en sortie des moyens de balayage est dirigé vers les moyens optiques 5 afin d'être injecté dans l'une des fibres du guide d'image 6. Ces moyens 5 sont constitués ici de deux ensembles optiques E1 et E2. Le premier ensemble optique E1 permet de corriger en partie les aberrations optiques en bord de champ des moyens de balayage 4, l'injection étant ainsi optimisée sur l'ensemble du champ optique (au centre comme au bord). Le second ensemble optique E2 est destiné à réaliser l'injection proprement dite. Sa focale et son ouverture numérique ont été choisies pour optimiser le taux d'injection dans les fibres optiques du guide 6. Selon un mode de réalisation permettant d'obtenir le critère d'achromaticité, le premier ensemble E1 est constitué d'un doublet de lentilles achromatiques, et le second ensemble E2 de deux doublets de lentilles achromatiques suivi d'une lentille située près du guide d'image. En variante, cette optique d'injection pourrait être constituée de tout autre type d'optiques standards, comme par exemple deux triplets, ou de lentilles à gradient d'indice ou bien d'un objectif de microscope (toutefois plus coûteux).

Le guide d'image 6 est constitué d'un très grand nombre de fibres optiques souples (quelques dizaines de milliers), par exemple 30 000 fibres de 2 µm de diamètre et espacées de 3,3 µm. En pratique, on peut utiliser soit l'ensemble des fibres du guide d'image, soit un sous-ensemble choisi de ces fibres, par exemple centré.

En sortie de la fibre optique, le faisceau laser d'excitation est focalisé par la tête optique 7 dans l'échantillon 13 en un point 14 situé à une profondeur donnée située entre quelques dizaines de µm et une centaine de µm, par rapport à la surface 15 de l'échantillon au contact de laquelle est destinée à être placée la tête optique. Cette profondeur peut être par exemple de 40 µm. La tête optique permet donc de focaliser le flux sortant du guide d'image dans l'échantillon, mais également de collecter le flux de fluorescence revenant de l'échantillon. La tête optique possède un grandissement de 2,4 et une ouverture numérique sur l'échantillon de 0,5. Ces deux paramètres sont choisis afin que le signal de retour se fasse uniquement dans la fibre optique ayant transmis le signal d'excitation et non pas dans des fibres adjacentes et conserver ainsi le filtrage confocal à l'aide d'une fibre. Avec ces valeurs de grandissement et d'ouverture numérique, la résolution axiale est de l'ordre de 10 µm et la résolution latérale de l'ordre de 1,4 µm. L'ouverture numérique est également choisie de manière à optimiser le nombre de photons récupérés qui doit être le plus important possible. La tête optique peut être constituée d'optiques classiques (doublet, triplet, asphérique) et/ou de lentilles à gradient d'indice (GRIN) et/ou de lentilles diffractives, présentant une qualité optique et un chromatisme adaptés à la confocalité, c'est à dire minimisant les aberrations optiques, qui entraîneraient sinon notamment des dégradations sur la profondeur de champ et par conséquent sur la résolution axiale de l'appareillage. En fonctionnement, la tête optique est destinée à être posée au contact de l'échantillon 13. Ce dernier est un tissu biologique ou une culture cellulaire. L'expression de la fluorescence est réalisée soit par un fluorophore que l'on injecte (fluorescence systémique), soit par un fluorophore fabriqué par la cellule elle même par modification d'un gêne (fluorescence transgénique). Dans ces deux cas, le fluorophore re-émet des photons sur une bande spectrale plus ou moins grande pouvant aller d'une dizaine de nanomètres à plus d'une centaine de nanomètres.

Sur la voie de détection, le signal de fluorescence, en sortie du filtre de réjection 8, est ensuite focalisé par les moyens 9, constitués par exemple d'une lentille de détection, dans un trou de filtrage des moyens 10 de filtrage spatial. La focale de la lentille de détection est calculée pour que le signal de fluorescence provenant d'une fibre soit de la taille ou légèrement inférieure à celle du trou de filtrage. Ce dernier permet de conserver la lumière de fluorescence ne provenant que de la fibre illuminée par le faisceau incident. Il permet de rejeter la lumière qui aurait pu être couplée dans les fibres adjacentes à celle qui est illuminée. La taille du trou est calculée pour que l'image d'une fibre s'y inscrive parfaitement. Ici, elle est de 20 µm. Par ailleurs, toujours dans un souci d'optimiser la quantité de photons traversant le trou de filtrage, et donc le flux détecté, les moyens de balayage 4, les moyens d'injection 5, les moyens de focalisation 7 de la tête optique, et les moyens de détection 8, 9 et 10 sont adaptés au fluorophore détecté : ces moyens sont choisis pour être suffisamment achromatiques pour collecter des photons sur la bande la plus large d'émission du fluorophore.

Les moyens de détection 11 ont une sensibilité maximale aux longueurs d'onde de fluorescence étudiées. On peut utiliser par exemple une photodiode à avalanches (APD) ou bien un photomultiplicateur. Par ailleurs, selon l'invention, la bande passante est choisie pour optimiser le temps d'intégration du signal de fluorescence. Elle est de 1,5 MHz, ce qui correspond à la fréquence d'échantillonnage minimale du guide d'image avec un temps d'intégration optimisé sur chaque pixel.

Les moyens électroniques et informatiques 12 de commande, d'analyse et de traitement numérique du signal détecté et de visualisation comprennent les cartes suivantes:
- une carte de synchronisation 20 qui a pour fonctions :
   - de commander de manière synchronisée le balayage, c'est-à-dire le mouvement des miroirs ligne M1 et trame M2 ;
   - de connaître à tout instant la position du spot laser ainsi balayé ; et
   - de gérer toutes les autres cartes par l'intermédiaire d'un micro-contrôleur lui-même pouvant être piloté ;
- une carte détecteur 21 qui comprend un circuit analogique qui réalise notamment une adaptation d'impédance, un convertisseur analogique numérique puis un composant logique programmable (par exemple un circuit FPGA) qui met en forme le signal ;
- une carte d'acquisition numérique 22 qui permet de traiter un flot de données numériques à fréquence variable et de l'afficher sur un écran 23 ;
- une carte graphique 24.

En variante, on peut utiliser une seule carte regroupant les fonctionnalités de ces différentes cartes.

Le traitement d'image se fait de la manière suivante.

A la mise en place d'un guide d'image dans l'appareillage, une première opération est effectuée pour reconnaître le motif des fibres dans le guide d'image, et donc connaître l'emplacement réel de chaque fibre destinée à être utilisée.

Les opérations suivantes sont également réalisées préalablement à l'utilisation de l'appareillage :
- la détermination du taux d'injection propre à chaque fibre, à l'aide d'un échantillon homogène, ce taux d'injection pouvant varier d'une fibre à une autre ; et
- la mesure de l'image de fond, effectuée sans échantillon.

Ces deux opérations peuvent être réalisées régulièrement en fonction de la fréquence d'utilisation de l'appareillage. Les résultats obtenus vont être utilisés pour corriger en fonctionnement le signal numérique en sortie de la carte détecteur.

En fonctionnement, selon l'invention 2 groupes de traitements sont effectués sur le signal numérique en sortie de la carte détecteur :

Le premier groupe consiste dans un premier temps à corriger le signal numérique notamment pour tenir compte du taux propre réel d'injection de la fibre dont est issu ledit signal et pour lui soustraire la partie du flux correspondant à l'image de fond. Cela permet de ne traiter qu'un signal correspondant réellement à l'échantillon observé. On utilise pour ce groupe de traitement un algorithme de calcul classique qui est optimisable pour respecter la contrainte du temps réel.

Le second groupe consiste ensuite, à partir du signal corrigé, à reconstruire l'image numérique qui sera visualisée par le praticien. Le but du traitement effectué est d'offrir à la visualisation une image numérique reconstituée qui ne soit pas simplement une mosaïque d'éléments d'image correspondant chacun à un signal numérique corrigé d'une fibre mis côte à côte, mais d'offrir une image numérique reconstituée qui ne fasse plus apparaître les fibres. On utilise pour cela un algorithme destiné à effectuer un certain nombre d'opérations sur chaque pixel, l'algorithme étant choisi pour respecter la contrainte de temps réel, c'est à dire qu'il doit représenter un bon compromis entre la complexité des opérations demandées, la qualité du résultat que l'on peut obtenir et le temps de calcul. A titre d'exemple, on peut utiliser un algorithme de filtrage passe-bas Gaussien.

Le fonctionnement de l'appareillage est le suivant. La source 1 produit un faisceau parallèle circulaire d'excitation à une longueur d'onde de 488 nm, qui est ensuite remis en forme dans le système afocal 2 afin de lui donner la taille adéquate pour la meilleure injection possible dans le coeur d'une fibre. Ce faisceau est ensuite envoyé vers le système de séparation dichroïque 3 qui réfléchit la longueur d'onde d'excitation. Le faisceau incident est ensuite dévié angulairement dans le temps dans les deux directions de l'espace par le système de balayage optomécanique de miroirs 4, et injecté grâce aux moyens optiques d'injection 5 dans l'une des fibres du guide d'image 6. Les moyens électroniques 12 servent à commander l'injection à un instant donné de l'une des fibres optiques du guide d'image en déviant angulairement le faisceau au moyen des miroirs, et ce point par point pour une ligne donnée, et ligne après ligne, pour constituer l'image. En sortie du guide, la lumière émergeant de la fibre injectée est focalisée dans l'échantillon grâce à la tête optique 7 en un point situé à une profondeur donnée située sensiblement entre quelques dizaines de µm et une centaine de µm. Grâce au balayage, l'échantillon est illuminé point par point. A chaque instant, le spot illuminant le tissu émet alors un signal de fluorescence qui a la particularité d'être décalé vers des plus grandes longueurs d'onde. Ce signal de fluorescence est capté par la tête optique 7, puis suit le chemin inverse du faisceau d'excitation jusqu'au filtre dichroïque 3 qui va transmettre le signal de fluorescence vers la voie de détection. Les réflexions parasites se produisant à la longueur d'onde d'excitation vont ensuite être rejetées par le filtre de réjection 8. Enfin, le signal de fluorescence est focalisé dans le trou de filtrage 10 pour ne sélectionner que la lumière provenant de la fibre excitée et les photons sont détectés par la photodiode à avalanche 11. Le signal détecté est ensuite numérisé et corrigé. Les signaux détectés, les uns après les autres, sont traités en temps réel grâce au traitement d'image décrit plus haut pour permettre la reconstruction d'une image en temps réel visualisée à l'écran.

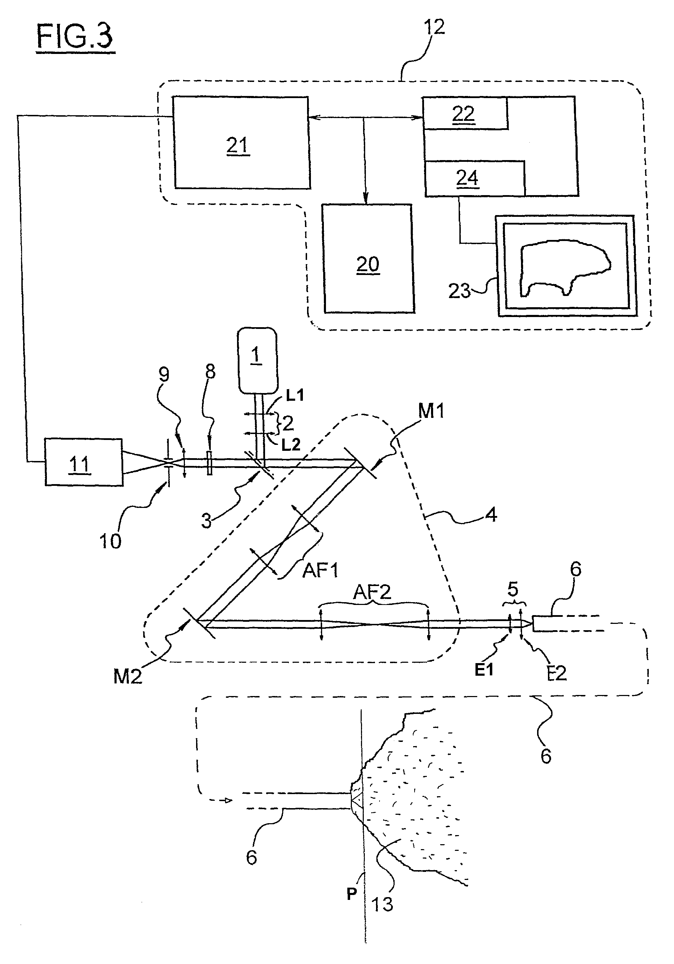

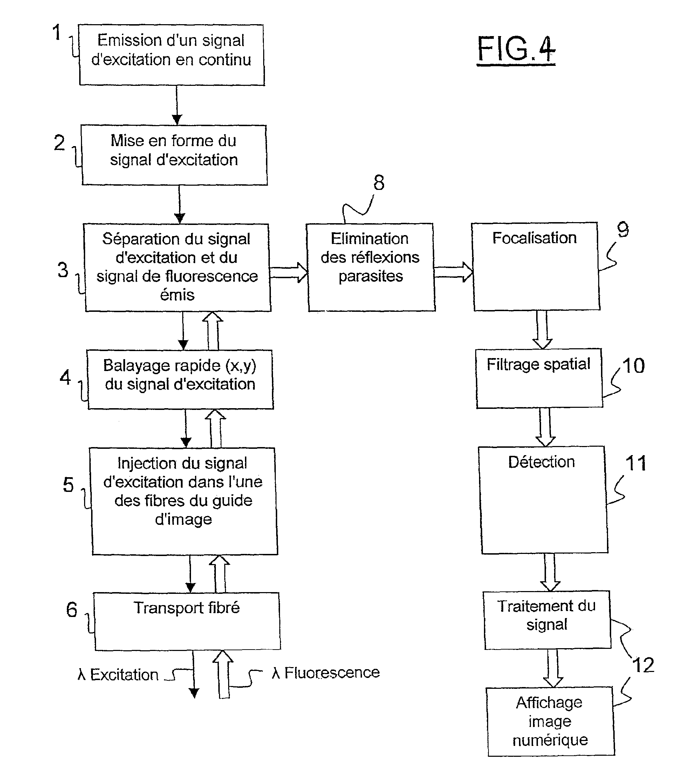

Selon l'exemple de réalisation choisi et représenté sur les figures 3 et 4, l'appareillage comporte les mêmes éléments que l'appareillage des figures 1 et 2 à l'exception de la tête optique de focalisation 7. Les mêmes références sont utilisées pour ces mêmes éléments sur les figures et ne seront donc pas redécrits ici en détails.

Le guide d'image 6 utilisé pour cet appareillage peut être composé sensiblement de 5 000 à 100 000 fibres optiques souples, selon le diamètre extérieur du guide que l'on souhaite, qui est lui même fonction de l'application visée (endoscopique, taille de champ recherché, etc.). Il peut s'agir par exemple de guides d'images commercialisés par la société FUJIKURA. Le diamètre de coeur des fibres est de préférence compris entre 1 et 4 µm. Cela conduit à une divergence de faisceau en sortie de la fibre présentant un angle d'environ 18° pour une ouverture numérique de 0,42 dans l'air. Un diamètre de coeur de 1µm peut être obtenu grâce à un procédé d'étirement de l'extrémité de l'ensemble du guide d'image. L'extrémité du guide 6 est polie et ne comporte pas de moyens optiques. Le polissage a pour but de conférer le même état de surface à la face du guide et donc aux fibres nues au contact du tissu pour, d'une part que le fond de l'image obtenu soit le plus homogène possible et, d'autre part, pour supprimer les éventuels problèmes d'adhérence des fibres avec le tissu qui risquerait de l'abîmer. Le polissage peut être plan ou arrondi pour épouser la forme du tissu. L'ouverture numérique de chaque fibre optique est de préférence choisie la plus grande possible pour collecter le maximum de photons de fluorescence, c'est à dire par exemple de 0,42. La distance inter-coeur confère la résolution latérale de l'image obtenue (distance entre deux points de l'image). Plus cette distance sera petite, meilleure sera la résolution, en revanche cela se fera au détriment de la taille du champ à imager. Il convient donc de trouver un bon compromis entre le nombre de fibres du guide et la distance inter-coeur entre les fibres pour obtenir une bonne résolution latérale (entre 2 et 8 µm) avec une taille de champ appropriée pour observer les éléments tissulaires souhaités.

Deux exemples de guides pouvant convenir selon l'invention sont donnés ci-après :

| | Exemple 1 | Exemple 2 |
|---|---|---|
| Nombre de fibres | 6 000 | 30 000 |
| Diamètre de champ imagé | 300 µm | 650 µm |
| Diamètre extérieur du guide | 330 µm | 750 µm |
| Diamètre de coeur d'une fibre | 3µm | 1,9µm |
| Distance inter-coeur | 4µm | 3,3 µm |
| Profondeur maximale d'intégration du signal depuis la surface (schématisé par le plan P sur la figure 1) ou résolution axiale | 15-20 µm | 10-15 µm |
| Résolution latérale | 4 µm | 3,3 µm |

En fonctionnement, l'extrémité distale du guide d'image est mise au contact de l'échantillon 13, la face d'extrémité des fibres étant ainsi directement au contact de la surface du tissu. Ce dernier est un tissu biologique ou une culture cellulaire. L'expression de la fluorescence est réalisée soit par un fluorophore que l'on injecte (fluorescence systémique), soit par un fluorophore fabriqué par la cellule elle même par modification d'un gêne (fluorescence transgénique). Dans ces deux cas, le fluorophore présent dans le micro-volume excité selon l'invention re-émet des photons sur une bande spectrale plus ou moins grande pouvant aller d'une dizaine de nanomètres à plus d'une centaine de nanomètres.

Le fonctionnement de l'appareillage est le même que celui décrit précédemment à l'exception de ce qui suit : en sortie du guide, la lumière divergente émergeant de la fibre injectée est diffusée dans l'échantillon et le signal de fluorescence est collecté dans un micro-volume situé entre la surface et une profondeur maximale de 25 µm (selon le diamètre de coeur des fibres et leur ON). Grâce au balayage, l'échantillon est illuminé micro-volume par micro-volume. A chaque instant, le micro-volume excité dans le tissu émet alors un signal de fluorescence qui a la particularité d'être décalé vers des plus grandes longueurs d'onde. Ce signal de fluorescence est capté par la même fibre optique que celle ayant servie à l'excitation, puis suit le chemin inverse du faisceau d'excitation jusqu'au filtre dichroïque 3 qui va transmettre le signal de fluorescence vers la voie de détection. Les signaux détectés, les uns après les autres, sont traités en temps réel grâce au même traitement d'image que celui décrit plus haut en référence aux figures 1 et 2 pour permettre la reconstruction d'une image en temps réel visualisée à l'écran.

## Revendications

1. Procédé pour réaliser une image de fluorescence haute résolution à l'aide d'un guide d'image fait de plusieurs milliers de fibres optiques, un signal d'excitation étant émis par une source continue, dévié et injecté tour à tour dans l'une des fibres optiques dudit guide d'image et un signal de fluorescence émis en réaction étant collecté par la même fibre optique que celle utilisée pour l'excitation, puis détecté et numérisé pour former un élément d'image, **caractérisé en ce que** l'extrémité des fibres est destinée à être placée à nue directement en contact de la surface du tissu à imager, chaque fibre étant adaptée à produire un faisceau divergent susceptible d'exciter un micro-volume du tissu situé de la surface jusqu'à une profondeur maximale dépendante notamment du diamètre de coeur des fibres optiques et **en ce que** l'on dévie le signal d'excitation à une vitesse correspondant à l'acquisition d'un nombre d'images par seconde suffisant pour une utilisation en temps réel et **en ce que** l'on détecte le signal de fluorescence à une fréquence de détection correspondant à une fréquence minimale d'échantillonnage des fibres une à une.

2. Procédé selon la revendication 1, **caractérisé en ce que** l'on règle une vitesse de déviation du faisceau d'excitation en déterminant une fréquence de résonance rapide d'un miroir résonnant ligne et une fréquence de résonance lente d'un miroir galvanométrique trame.

3. Procédé selon l'une des revendications précédentes, **caractérisé en ce que** l'on utilise des moyens optiques de déviation, d'injection, de focalisation et de détection présentant un degré d'achromaticité permettant de collecter des photons sur toute la largeur de la bande d'émission du fluorophore excité.

4. Procédé selon l'une des revendications précédentes, **caractérisé par** une efficacité quantique de la détection aux longueurs d'ondes de fluorescence à détecter d'au moins 50%.

5. Procédé selon l'une des revendications précédentes, **caractérisé par** une étape préalable de détection de l'emplacement des fibres du guide d'image destinées à être utilisées.

6. Procédé selon l'une des revendications précédentes, **caractérisé par** une étape préalable de détermination du taux réel d'injection propre à chaque fibre.

7. Procédé selon l'une des revendications précédentes, **caractérisé par** une étape préalable de détermination du flux collecté correspondant à l'image de fond.

8. Procédé selon les revendications 6 et 7, **caractérisé par** une étape de correction du signal numérisé en provenance d'une fibre par soustraction du flux correspondant à l'image de fond et adaptation au taux réel d'injection propre de ladite fibre.

9. Procédé selon la revendication 8, **caractérisé par** une étape de reconstruction de l'image à partir du signal corrigé.

10. Procédé selon la revendication 9, **caractérisé en ce que** l'étape de reconstruction d'image comprend un filtrage passe- bas Gaussien.

11. Procédé selon l'une quelconque des revendications précédentes, **caractérisé en ce que** le signal d'excitation est adapté à un fluorophore de type endogène du tissu cellulaire.

12. Appareillage d'imagerie de fluorescence fibrée in vivo in situ haute résolution pour la mise en oeuvre du procédé selon l'une des revendications précédentes, comprenant :
- le guide d'image (6) ;
- la source (1) émettant en continu à la longueur d'onde d'excitation d'au moins un fluorophore ciblé,
- des moyens de balayage (4) et d'injection (5) fibre après fibre du faisceau d'excitation produit par la source (1) dans un plan XY correspondant à la section d'entrée du guide d'image (6);
- des moyens (3) de séparation de la longueur d'onde d'excitation et des longueurs d'onde de fluorescence ;
- des moyens de détection (11) du signal de fluorescence ; et
- des moyens (12) de traitement du signal détecté permettant la réalisation d'une image ;
**caractérisé en ce que** l'extrémité de chaque fibre est adaptée à produire un faisceau qui est divergent et est destinée à être placée à nue directement au contact de la surface du tissu à observer ;
et **en ce que** les moyens de balayage sont adaptés à déplacer le faisceau d'excitation à une vitesse correspondant à l'obtention d'une image en temps réel et les moyens de détection présentent une bande passante dont la fréquence est fixée en fonction de la fréquence minimale d'échantillonnage des fibres une à une.

13. Appareillage selon la revendication 12, **caractérisé en ce que** le faisceau d'excitation produit par la source (1) est monomode longitudinal présentant une qualité de front d'onde optimale pour l'injection dans une fibre optique faiblement multimode.

14. Appareillage selon l'une des revendications 12 et 13, **caractérisé en ce que** la section d'une fibre étant circulaire, le faisceau d'excitation produit par la source est circulaire de manière à optimiser l'injection dans une fibre.

15. Appareillage selon l'une des revendications 12-14, **caractérisé par** des moyens (2) de mise en forme du faisceau utilisés en sortie de la source (1) pour mettre en forme le faisceau d'excitation afin de l'adapter aux moyens d'injection (5) dans le guide d'image (6).

16. Appareillage selon l'une des revendications 12-15, **caractérisé en ce que** les moyens pour séparer les longueurs d'onde d'excitation et de fluorescence comprennent un filtre dichroïque (3) ayant un maximum d'efficacité à la longueur d'onde d'excitation.

17. Appareillage selon l'une des revendications 12-16, **caractérisé par** des moyens de réjection (8) placé en amont des moyens de détection (11) et adaptés à éliminer la longueur d'onde d'excitation.

18. Appareillage selon l'une des revendications 12-17, **caractérisé en ce que** les moyens de balayage (4) comprennent un miroir ligne résonnant (M1), un miroir galvanométrique trame (M2), un premier système optique afocal de grandissement unitaire (AF1) permettant de conjuguer les deux miroirs et un second système afocal (AF2) de grandissement unitaire permettant de conjuguer les plans de rotation des deux miroirs avec le plan d'injection dans l'une des fibres.

19. Appareillage selon l'une des revendications 12-18, **caractérisé en ce que** les moyens optiques de la tête optique (7), les moyens de balayage (4), les moyens d'injection 5) et les moyens de détection présentent un degré d'achromatisation permettant de collecter des photons sur toute la largeur de la bande spectrale du signal de fluorescence.

20. Appareillage selon l'une des revendications 12-19, **caractérisé en ce que** les moyens d'injection (5) comprennent deux ensembles optiques (E1,E2), le premier ensemble (E1) étant adapté à corriger les aberrations optiques en bordure de champ des moyens de balayage (4) et le second ensemble (E2) étant adapté à réaliser l'injection proprement dite dans l'une des fibres du guide d'image (6).

21. Appareillage selon la revendication 20, **caractérisé en ce que** le premier ensemble optique (E1) comprend un doublet et le second ensemble optique (E2) comprend deux doublets suivi d'une lentille.

22. Appareillage selon l'une des revendications 12-21, **caractérisé par** un trou de filtrage (10) placé avant les moyens de détection (11) dont le diamètre est choisi pour que l'image d'une fibre s'y inscrive.

23. Appareillage selon la revendication 22, **caractérisé par** des moyens de focalisation (9) du signal de fluorescence sur le trou de filtrage (10).

24. Appareillage selon l'une des revendications 12-23, **caractérisé en ce que** la source est adaptée à exciter un fluorophore endogène présent dans le tissu cellulaire.

25. Guide d'image pour la mise en oeuvre du procédé selon l'une des revendications 1 à 11 et pour la mise en oeuvre de l'appareillage selon l'une des revendications 12 à 24, **caractérisé en ce qu'**il comporte une extrémité distale où les fibres sont nues.

26. Guide d'image selon la revendication 25, **caractérisé en ce que** son extrémité distale est polie de manière que le fond de l'image obtenue soit le plus homogène possible.

27. Guide d'image selon la revendication 26, **caractérisé en ce que** le polissage est plan.

28. Guide d'image selon la revendication 26, **caractérisé en ce que** le polissage est arrondi.
